# EUROPÄISCHE PATENTSCHRIFT

(11) **EP 3 931 296 B1**
(45) Veröffentlichungstag und Bekanntmachung des Hinweises auf die Patenterteilung: **28.12.2022**
(21) Anmeldenummer: 20701698.1
(22) Anmeldetag: 15.01.2020
(51) Int. Cl.: C12M 1/00, C12M 3/00, C12M 1/06, C12M 1/34

(54) **SENSORPOSITIONIERSYSTEM FÜR EINE BIOPROZESSTECHNISCHE ANLAGE**
SENSOR POSITIONING SYSTEM FOR A BIOPROCESS ENGINEERING INSTALLATION
SYSTÈME DE POSITIONNEMENT DE CAPTEUR POUR UNE INSTALLATION DE BIOPROCÉDÉS

(30) Priorität: 25.03.2019 DE 102019107622
(43) Veröffentlichungstag der Anmeldung: 05.01.2022
(73) Patentinhaber: Sartorius Stedim Biotech GmbH, 37079 Göttingen (DE)
(72) Erfinder: ADAMS, Thorsten, 37085 Göttingen (DE); RUPPRECHT, Jens, 31139 Hildesheim (DE); GRIMM, Christian, 37308 Heilbad Heiligenstadt (DE); BODE, Matthias, 37434 Krebeck (DE)
(74) Vertreter: Vigand, Philippe
(86) Internationale Anmeldenummer: PCT/EP2020/050921
(87) Internationale Veröffentlichungsnummer: WO 2020/192976

(56) Entgegenhaltungen:
- DE-A1-102010 060 131
- DE-A1-102013 109 820
- DE-A1-102015 007 060
- US-A1- 2013 101 982
- US-B2- 9 975 658

## Beschreibung

Die Erfindung betrifft ein Sensorpositioniersystem für eine bioprozesstechnische Anlage gemäß dem Oberbegriff von Anspruch 1, ein Sensorpositioniersystem für eine bioprozesstechnische Anlage gemäß dem Oberbegriff von Anspruch 13, eine bioprozesstechnische Anlage mit einem solchen Sensorpositioniersystem gemäß Anspruch 14 sowie ein Verfahren zur Positionierung eines Sensors einer bioprozesstechnischen Anlage gemäß dem Oberbegriff von Anspruch 15.

Unter einer bioprozesstechnischen Anlage wird hier ganz allgemein eine Einrichtung verstanden, mit der biotechnologische Prozesse durchgeführt oder unterstützt werden können. Lediglich beispielhaft seien hier Bioreaktoren genannt, in denen Mikroorganismen oder Gewebezellen unter vorgegebenen Bedingungen kultiviert werden. Als weiteres Beispiel bioprozesstechnischer Anlagen seien hier Prozess-Mischsysteme genannt, die zum Mischen von Pulvern, Suspensionen, Lösungen oder Emulsionen dienen können, die in biotechnologischen Prozessen eingesetzt werden. Solche Einrichtungen weisen regelmäßig einen Container auf, in welchem ein biologisches Medium aus den für einen biotechnologischen Prozess vorgesehenen Stoffen, zum Beispiel Mikroorganismen oder Gewebezellen einerseits und ein entsprechendes Nährmedium andererseits, aufgenommen wird, um den jeweiligen biotechnologischen Verfahrensschritt, beispielsweise eine Fermentierung bzw. Kultivierung und/oder ein Durchmischen durchführen zu können. Das Erzeugnis, bei einem Bioreaktor, beispielsweise die Fermentationsbrühe, wird dann üblicherweise in einem sogenannten Downstream-Prozess weiterbehandelt, um ein Produkt aus den Zellen oder dem Kulturüberstand zu erhalten.

Als Container für eine solche bioprozesstechnische Anlage kann einerseits ein formstabiles Gefäß, andererseits aber auch ein flexibler, das heißt biegeschlaffer, Beutel, auch als Bioprozessbeutel bezeichnet, verwendet werden. Sowohl das formstabile Gefäß, als auch der Bioprozessbeutel sind dabei dem allgemeinen Trend folgend vorzugsweise als Einwegcontainer (Single-Use-Container) ausgestaltet. Außerdem kann ein solcher Container bereits mit einem integrierten Rührwerk versehen sein, mit dem das biologische Medium durchmischt werden kann. Im Rahmen des Kultivierens und/oder durch das Durchmischen entsteht dabei oberhalb des Flüssigkeitspegels oftmals Schaum als Störgröße. Dabei besteht häufig das Risiko, dass der Schaum in den Abluftfilter der bioprozesstechnischen Anlage, beispielsweise des Bioreaktors, steigt und zu einem Verblocken des Filters führt. Der daraus resultierende, sehr schnelle Druckanstieg in der bioprozesstechnischen Anlage macht die weitere Regelung des Sauerstoffgehalts des biologischen Mediums im Container nahezu unmöglich. In der Folge muss daher schlimmstenfalls ein vollständiger Batch verworfen werden. Dies kann gerade im pharmazeutischen Umfeld einen Produktionsausfall durchaus im siebenstelligen Bereich verursachen.

Aus dem Stand der Technik (DE 10 2013 109 820 A1), von dem die Erfindung ausgeht, ist es daher bekannt, zur Detektion von Schaum als Störgröße Füllstandsensoren zu verwenden. Diese sind hier als sogenannte Füllstandgrenzschalter ausgestaltet und im Inneren des Containers mit dem biologischen Medium angeordnet. Dabei ist zur Anpassung der bioprozesstechnischen Anlage an unterschiedliche bioprozesstechnische Anwendungsfälle ein Sensorpositioniersystem vorgesehen, das es erlaubt, den Füllstandsensor in unterschiedliche Höhenstellungen zu bringen. So weist der Sensor hier zwei elektrisch leitfähige Plättchen auf, die im Container in unterschiedlicher Höhe angeordnet sind und zwischen denen die Leitfähigkeit oder Impedanz des die Plättchen umgebenden Schaums ermittelt wird. Das jeweils höhere, also vom Flüssigkeitspegel weiter entfernte, Plättchen ist dabei in Axialrichtung des Containers bzw. hier in der Vertikalen in unterschiedlichen Positionen anordenbar.

Das Vorsehen des Füllstandsensors in der für einen spezifischen bioprozesstechnischen Anwendungsfall optimalen Höhenstellung ist dabei dem Geschick und der Erfahrung des Benutzers, der die bioprozesstechnische Anlage einrichtet, überlassen. Ein zuverlässiges standardisiertes Verfahren für eine anwendungsspezifisch optimale Positionierung eines Füllstandsensors existiert bisher nicht.

Der Erfindung liegt das Problem zugrunde, ein Sensorpositioniersystem für eine bioprozesstechnische Anlage derart auszugestalten und weiterzubilden, dass auf möglichst einfache Weise eine für einen spezifischen bioprozesstechnischen Anwendungsfall optimale Positionierung eines Füllstandsensors in einem Container der bioprozesstechnischen Anlagen erfolgen kann.

Das obige Problem wird bei einem Sensorpositioniersystem für eine bioprozesstechnische Anlage gemäß dem Oberbegriff von Anspruch 1 durch die Merkmale des kennzeichnenden Teils von Anspruch 1 gelöst.

Wesentlich ist die grundsätzliche Überlegung, eine Positionierhilfseinrichtung vorzusehen, über die dem Benutzer die für einen spezifischen bioprozesstechnischen Anwendungsfall optimale Position zur Fixierung eines insbesondere aufklebbaren Sensors, beispielsweise eines Füllstandsensors, insbesondere eines Schaumsensors, angezeigt wird. Zu diesem Zweck ist eine Positionierhilfe vorgesehen, die relativ zu einem definierten Bezugspunkt an der bioprozesstechnischen Anlage in eine für den jeweiligen Anwendungsfall vorgegebene Höhenstellung und/oder Umfangsstellung gebracht wird und an der der zu fixierende Sensor anschließend ausgerichtet werden kann. Dabei sind Höhenstellungen Stellungen relativ zum Container in Höhenrichtung, also in Verlaufsrichtung der axialen Mittelachse des Containers, die sich üblicherweise vertikal erstreckt. Umfangsstellungen sind Stellungen relativ zum Container in Umfangsrichtung, also um die axiale Mittelachse des Containers herum. In der mittels der Positionierhilfe dem Benutzer angezeigten bzw. vorgegebenen Position kann dann der Sensor am Container fixiert werden.

Die jeweils korrekte Höhen- und/oder Umfangsstellung der Positionierhilfe wird dabei über eine Zuordnungsliste ermittelt, die für mehrere Anwendungsfälle jeweils eine zugeordnete Höhen- und/oder Umfangsstellung beinhaltet. Die Höhen- bzw. Umfangsstellungen können in der Zuordnungsliste als Abstände zu dem jeweiligen Bezugspunkt und/oder als feste Positionen, die über die Positionierhilfe relativ zu dem jeweiligen Bezugspunkt anzeigbar sind, abgelegt sein. Aus der Zuordnungsliste ergibt sich also genau eine eindeutige Höhen- und/oder Umfangsstellung, in die die Positionierhilfe für einen spezifischen Anwendungsfall gebracht werden muss, wobei die Positionierhilfe dann in dieser Höhen- und/oder Umfangsstellung dem Benutzer die zugehörige Position für den Sensor anzeigt, in der diese am Container fixiert werden muss.

Im Einzelnen wird nun vorgeschlagen, dass eine Positionierhilfseinrichtung zur Positionierung des zu fixierenden Sensors in einer vorgegebenen Position am Container vorgesehen ist, dass die Positionierhilfseinrichtung eine unmittelbar am Container oder neben dem Container am Halter angebrachte oder anbringbare Positionierhilfe aufweist, die relativ zu mindestens einem am Container und/oder Halter vorgesehenen Bezugspunkt in verschiedene Höhen- und/oder Umfangsstellungen bringbar ist, und dass die Positionierhilfseinrichtung ein technisches Speichermedium oder analoges Druckmedium mit einer Zuordnungsliste aufweist, in der mehreren bioprozesstechnischen Anwendungsfällen jeweils eine vorgegebene Höhen- und/oder Umfangsstellung der Positionierhilfe zugeordnet ist.

Ein "technisches Speichermedium", auch als Datenträger bezeichnet, kann ganz allgemein ein elektronisches, magnetisches, optisches oder fotografisches Speichermedium zur Speicherung der Zuordnungsliste sein. Ein "analoges Druckmedium" kann ein Medium in Papierform oder Folienform zur Speicherung der Zuordnungsliste sein, zum Beispiel mit der Zuordnungsliste bedrucktes oder beschriebenes Papier bzw. bedruckte oder beschriebene Folie.

Die Ansprüche 2 und 3 betreffen besonders bevorzugte Ausgestaltungen des Speicher- oder Druckmediums und der Zuordnungsliste.

Die Ansprüche 4 bis 6 geben besonders bevorzugte Möglichkeiten an, wie die Positionierhilfe in die jeweiligen Höhenstellungen und Umfangsstellungen bringbar ist. Insbesondere kann die Positionierhilfe als Ganzes in Axialrichtung, also entlang der Mittelachse des Containers, und/oder in Umfangsrichtung in der sich aus der Zuordnungsliste ergebenden Stellung, das heißt der Höhenstellung und/oder Umfangsstellung angeordnet werden. Grundsätzlich ist es aber auch denkbar, dass nur ein bewegliches Teil der Positionierhilfe in die jeweilige Höhenstellung und/oder Umfangsstellung gebracht wird, wohingegen die Positionierhilfe im Übrigen ortsfest ist.

In den Ansprüchen 7 und 8 sind besonders bevorzugte Ausgestaltungen der Positionierhilfe definiert. Diese stellt dabei besonders bevorzugt mindestens eine Markierung bereit, an der der Sensor ausrichtbar ist. Ein Bereitstellen einer Markierung kann zum einen darin bestehen, dass die Positionierhilfe mindestens eine Markierung aufweist, also beispielsweise eine Markierung auf dieser aufgebracht ist. Zum anderen kann die Positionierhilfe die Markierung aber auch selbst bilden, das heißt die Positionierhilfe als solche ist gleichzeitig auch die Markierung.

Anspruch 9 definiert besonders bevorzugte Anordnungen der Positionierhilfe und des Speicher- oder Druckmediums relativ zum Sensorpositioniersystem im Übrigen.

In Anspruch 10 sind besonders bevorzugte Ausgestaltungen des Containers angegeben, der vorzugsweise ein Bioprozessbeutel oder ein formstabiles Gefäß ist. Insbesondere handelt es sich bei dem Container um einen Einwegcontainer. Der Container kann auch ein integriertes Rührwerk aufweisen.

Anspruch 11 gibt besonders bevorzugte Bezugspunkte an, relativ zu denen die Positionierhilfe in die verschiedenen Höhenstellungen und/oder Umfangsstellungen bringbar ist.

Anspruch 12 definiert besonders bevorzugte Ausgestaltungen des Sensors, der beispielsweise ein Füllstandsensor ist. Aber auch Sensoren zum Ermitteln anderer physikalischer Parameter sind denkbar. Der Begriff "Füllstandsensor" ist hier weit zu verstehen und umfasst sowohl Füllstandgrenzschalter, also Sensoren zur Überwachung von Grenzständen, als auch Sensoren zur kontinuierlichen Messung des Füllstandes. Mit einem Füllstandsensor lässt sich allgemein der Übergang der Grenzschicht zwischen zwei Medien (z.B. Schaum/Flüssigkeit oder Flüssigkeit/Flüssigkeit) und/oder Aggregatzuständen (z.B. gasförmige Phase/flüssige Phase) ermitteln bzw. verfolgen. Das Medium kann auch ein Zwei- oder Dreiphasengemisch sein.

Nach einer weiteren Lehre gemäß Anspruch 13, der eigenständige Bedeutung zukommt, wird ein Sensorpositioniersystem für eine bioprozesstechnische Anlage beansprucht, bei der die Positionierhilfe der Positionierhilfseinrichtung vorzugsweise nicht höhenverstellbar und nicht in Umfangsrichtung verstellbar ist, sondern unmittelbar auf dem Container vorgesehen ist. Eine solche Positionierhilfe kann beispielsweise bereits werksseitig auf den Container, beispielsweise als Skala, aufgedruckt oder aufgeklebt sein. Gemäß dieser weiteren Lehre wird unter Zuhilfenahme der Zuordnungsliste der Sensor selbst in eine vorgegebene Höhen- und/oder Umfangsstellung gebracht, wohingegen die Positionierhilfe insbesondere ortsfest ist. Dabei sind Höhenstellungen Stellungen relativ zur Positionierhilfe in Höhenrichtung, also in Verlaufsrichtung der axialen Mittelachse des Containers, die sich üblicherweise vertikal erstreckt. Umfangsstellungen sind Stellungen relativ zur Positionierhilfe in Umfangsrichtung, also um die axiale Mittelachse des Containers herum. Im Übrigen darf hinsichtlich des vorschlagsgemäßen Sensorpositioniersystems der weiteren Lehre auf alle Ausführungen zu der erstgenannten Lehre verwiesen werden

Nach einer weiteren Lehre gemäß Anspruch 14, der ebenfalls eigenständige Bedeutung zukommt, wird eine bioprozesstechnische Anlage, insbesondere ein Bioreaktor oder Prozess-Mischsystem, mit einem vorschlagsgemäßen Sensorpositioniersystem beansprucht. Angesichts der Tatsache, dass die vorschlagsgemäße, bioprozesstechnische Anlage ein vorschlagsgemäßes Sensorpositioniersystem aufweist, darf auf alle diesbezüglichen Ausführungen zu der erstgenannten Lehre verwiesen werden.

Nach einer weiteren Lehre gemäß Anspruch 15, der ebenfalls eigenständige Bedeutung zukommt, wird ein Verfahren zur Positionierung eines Sensors einer bioprozesstechnischen Anlage, insbesondere unter Verwendung eines vorschlagsgemäßen Sensorpositioniersystems, beansprucht, wobei ein Container, insbesondere Einwegcontainer, zur Aufnahme eines biologischen Mediums bereitgestellt wird, wobei der Container von einem Halter aufgenommen wird, der den Container hält, derart, dass sich der Container in Axialrichtung von einem oberen Containerende zu einem unteren Containerende erstreckt. Das vorschlagsgemäße Verfahren ist dadurch gekennzeichnet, dass nach der Aufnahme des Containers durch den Halter der Sensor an einer Positionierhilfe einer Positionierhilfseinrichtung ausgerichtet wird und am Container fixiert wird, wobei die Ausrichtung basierend auf einer vorgegebenen Höhen- und/oder Umfangsstellung erfolgt, die in einer Zuordnungsliste eines technischen Speichermediums oder analogen Druckmediums einem von mehreren bioprozesstechnischen Anwendungsfällen zugeordnet ist. Mit dem vorschlagsgemäßen Verfahren lassen sich auch mehrere Sensoren, insbesondere unterschiedliche Sensoren, ausrichten und in der für den jeweiligen Anwendungsfall vorgegebenen Position am Container 3 fixieren. Es darf hinsichtlich des vorschlagsgemäßen Verfahrens auf alle Ausführungen zu den weiteren Lehren verwiesen werden.

Die Ansprüche 16 und 17 betreffen besonders bevorzugte Ausgestaltungen des Verfahrens. So kann einerseits die Positionierhilfe in unterschiedliche Höhenstellungen und/oder Umfangsstellungen gebracht werden, an denen dann der Sensor ausgerichtet wird (Anspruch 16). Andererseits kann aber auch der Sensor relativ zu der Positionierhilfe in verschiedene Höhenstellungen und/oder Umfangsstellungen gebracht werden und dann an der Positionierhilfe ausgerichtet werden (Anspruch 17). In letzterem Fall ist die Positionierhilfe bereits vor der Aufnahme des Containers durch den Halter unmittelbar am Container oder Halter angebracht, beispielsweise aufgedruckt oder aufgeklebt, wobei die Positionierhilfe insbesondere fest, das heißt unbeweglich, ist, also selbst nicht in unterschiedliche Höhenstellungen bzw. Umfangsstellungen gebracht werden kann. Grundsätzlich wäre aber auch der Fall denkbar, dass zunächst, vor oder nach dem Anbringen der Positionierhilfe, diese in eine bestimmte Höhenstellung und/oder Umfangsstellung relativ zu einem Bezugspunkt am Container und/oder Halter gebracht wird und anschließend der Sensor relativ zu der solchermaßen eingestellten Positionierhilfe in eine vorgegebene Höhenstellung und/oder Umfangsstellung gebracht wird. Die Höhenstellung bzw. Umfangsstellung der Positionierhilfe und die Höhenstellung bzw. Umfangsstellung des Sensors sind dann in der Zuordnungsliste einem spezifischen bioprozesstechnischem Anwendungsfall zugeordnet.

Im Folgenden wir die Erfindung anhand einer lediglich Ausführungsbeispiele darstellenden Zeichnung näher erläutert. In der Zeichnung zeigt
- Fig. 1: eine vorschlagsgemäße, bioprozesstechnische Anlage mit einem vorschlagsgemäßen Sensorpositioniersystem,
- Fig. 2: eine schematische Darstellung der Positionierung eines Sensors der bioprozesstechnischen Anlage gemäß Fig. 1 mittels eines vorschlagsgemäßen Sensorpositioniersystems gemäß einem ersten Ausführungsbeispiel und
- Fig. 3: eine schematische Darstellung der Positionierung eines Sensors mittels eines vorschlagsgemäßen Sensorpositioniersystems gemäß einem weiteren Ausführungsbeispiel.

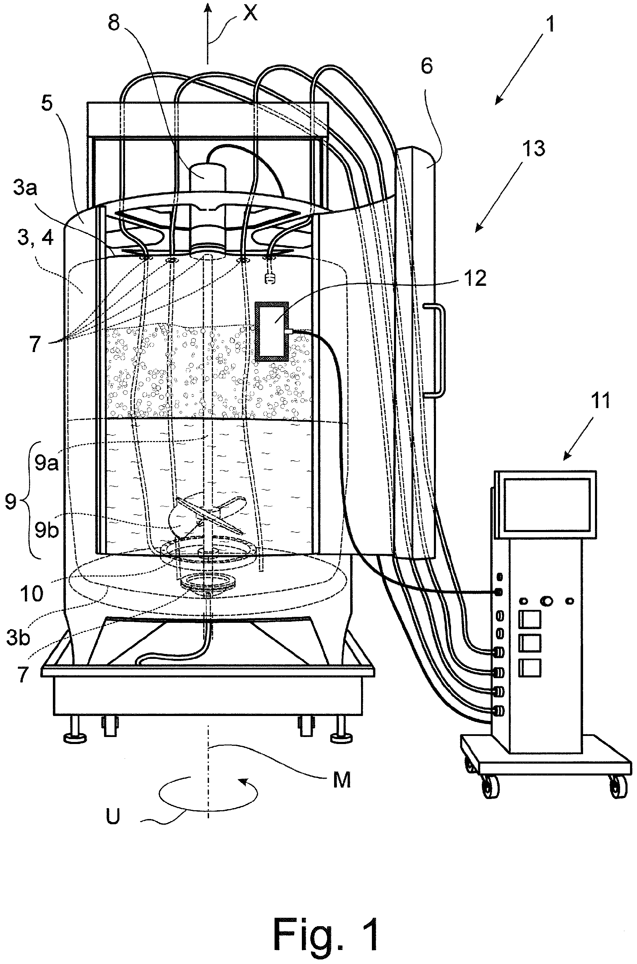

Fig. 1 zeigt schematisch eine bioprozesstechnische Anlage 1 in Form eines Bioreaktors 2. Der Bioreaktor 2 weist einen Container 3, insbesondere Einwegcontainer, zur Aufnahme eines biologischen Mediums, hier eines biologischen Reaktionsmediums, auf, wobei das Reaktionsmedium insbesondere Mikroorganismen wie Bakterien, Hefen, Viren o.dgl. oder Gewebezellen wie Tierzellen, Pflanzenzellen o.dgl. sowie ein Nährmedium aufweist. Der Container 3 ist hier als Bioprozessbeutel 4 ausgestaltet, also als Container mit zumindest abschnittsweise flexiblen Wänden. Grundsätzlich kann der Container 3 in einer alternativen, hier nicht dargestellten Ausführungsform auch als formstabiles Gefäß ausgestaltet sein.

Zur Bildung eines mikrobiellen oder zellulären Produkts erfolgt in dem Container 3 unter vorgegebenen Bedingungen eine Kultivierung der Mikroorganismen bzw. Gewebezellen. Der Container 3 erlaubt es, neben den Mikroorganismen bzw. Gewebezellen einerseits und dem Nährmedium andererseits noch weitere flüssige oder gasförmige Medien, wie Sauerstoff, pH-Korrekturmittel o.dgl. zuzuführen und die durch die Kultivierung erfolgte Fermentationsbrühe und gegebenenfalls sich im Kopfraum des Containers 3 ansammelnde Gase jeweils abzuführen. Die abgeführte Fermentationsbrühe kann dann anschließend zur Gewinnung des Produkts unter anderem einem Filter oder einer Zentrifuge zugeführt werden.

Ein weiteres, hier nicht dargestelltes Ausführungsbeispiel einer bioprozesstechnischen Anlage 1 ist ein Prozess-Mischsystem, das zum Mischen von Pulvern, Suspensionen, Lösungen oder Emulsionen dient, die in biotechnologischen Prozessen, beispielsweise in einem Downstream- oder Upstream-Prozess, eingesetzt werden können.

Die vorschlagsgemäße, bioprozesstechnische Anlage 1, hier in Form des Bioreaktors 2, weist ferner einen Halter 5, hier in Form eines Edelstahlgehäuses, auf, in welchem der Container 3, hier der Einwegcontainer, installiert wird. Im installierten Zustand erstreckt sich der Container 3 in Axialrichtung X, hier in der Vertikalen bzw. Schwerkraftrichtung, von einem oberen Containerende 3a zu einem unteren Containerende 3b. Mit einem Halter 5 ist ganz allgemein eine Einrichtung der bioprozesstechnischen Anlage 1 gemeint, die den Container 3 im installierten Zustand aufnimmt und dadurch hält. Der Halter 5 kann, wie hier das Edelstahlgehäuse, den Container 3 zumindest teilweise, hier und vorzugsweise vollständig, umgeben und den Container 3 entsprechend in seinem Inneren aufnehmen, um den Container 3 im Betrieb der bioprozesstechnischen Anlage 1 zu halten und mechanisch zu stabilisieren. Hier und vorzugsweise weist der Halter 5 ferner eine Tür 6 auf, die hier um eine zur Axialrichtung X parallele Schwenkachse zwischen der in Fig. 1 schematisch dargestellten Offenstellung und einer nicht dargestellten Schließstellung schwenkbar ist. In der Schließstellung der Tür 6 weist der Halter 5 hier insgesamt eine im Wesentlichen zylindrische Form mit einer sich in Axialrichtung X erstreckenden Zylinderachse auf. Der Container 3, hier in Form des Bioprozessbeutels 4, hat hier und vorzugsweise eine korrespondierende Form, die so gewählt ist, dass der Container 3 im bestimmungsgemäß installierten Zustand mit seiner Außenseite innenseitig an der Wand des Halters 5 bzw. Edelstahlgehäuses zur Anlage kommt.

Der Container 3 weist hier und vorzugsweise mehrere Anschlussstücke 7 auf, die hier uns vorzugsweise jeweils zur Aufnahme bzw. zum Anschluss eines pneumatischen und/oder hydraulischen Leitungsstücks und/oder eines Sensors bzw. einer Sonde und/oder zum Anschluss eines Antriebsmotors 8 dienen. Einige der Anschlussstücke 7 sind hier in der oberen Containerhälfte, insbesondere im Bereich des oberen Containerendes 3a, und ein weiteres der Anschlussstücke 7 in der unteren Containerhälfte, insbesondere im Bereich des unteren Containerendes 3b, angeordnet. Letzteres bildet beispielsweise einen Bodenablass zum Abführen von Fermentationsbrühe. Die weiteren Anschlussstücke 7 können Anschlussstücke zur Einleitung von gasförmigen und/oder flüssigen Medien in das Containerinnere und/oder zum Abführen von insbesondere gasförmigen Medien aus dem Containerinneren, sowie ein pO₂-Anschlussstück zum Anschluss eines pO₂- bzw. DO-Sensors, ein pH-Sensor-Anschlussstück zum Anschluss eines pH-Sensors, ein Temperatursensor-Anschlussstück zum Anschluss eines Temperatursensors oder ein Probenahme-Anschlussstück zum Anschluss einer Probenahme-Sonde umfassen.

Der Antriebsmotor 8 dient hier und vorzugsweise zum Antrieb eines optionalen Rührwerks 9, das hier in den Container 3 integriert ist. Das Rührwerk 9 weist hier und vorzugsweise eine Rührwelle 9a sowie mindestens ein Rührorgan 9b, hier in Form eines Propellerrührers, auf. Das Rührwerk 9 dient zur Durchmischung der biologischen Reaktionsbrühe während der Kultivierung. In Axialrichtung X unterhalb des hier einzigen Rührorgans 9b ist in Fig. 1 ferner eine optionale Begasungseinrichtung 10 dargestellt, die hier und vorzugsweise zur Zufuhr von Sauerstoff in das Reaktionsmedium dient.

Die Anschlussstücke 7 können, wie in Fig. 1 ebenfalls beispielhaft dargestellt ist, über elektrische und/oder hydraulische und/oder pneumatische Leitungsabschnitte mit einer Steuereinrichtung 11 zur Prozesssteuerung verbunden sein, die ebenfalls Teil der bioprozesstechnischen Anlage 1 ist.

Ferner weist die bioprozesstechnische Anlage 1, wie Fig. 1 ebenfalls zeigt, einen, insbesondere außenseitig, am Container 3 fixierten Sensor 12 auf. Besonders bevorzugt handelt es sich bei dem Sensor 12 um einen induktiven Sensor, einen kapazitiven Sensor, einen optischen Sensor, einen Ultraschallsensor, einen Mikrowellensensor, einen Radarsensor oder einen Sensor zur Messung der elektrischen Leitfähigkeit oder Wärmeleitfähigkeit. Der Sensor 12 ist hier und vorzugsweise ein Füllstandsensor und dient insbesondere zur kontinuierlichen Füllstandmessung. Alternativ kann auch ein Füllstandsensor vorgesehen sein, der zur Grenzstandmessung dient. In letzterem Fall ist der Füllstandsensor dann als Füllstandgrenzschalter ausgestaltet. Bei dem Füllstandsensor handelt es sich hier und vorzugsweise um einen Schaumsensor, der eine kontinuierliche Verfolgung der Schaumhöhe von sich oberhalb des Flüssigkeitspegels bildendem Schaum erlaubt. Der zu ermittelnde Füllstand ist entsprechend durch die Oberseite der Schaumschicht definiert. Der Füllstandsensor ist hier lediglich beispielhaft genannt. Grundsätzlich muss als Sensor 12 für die bioprozesstechnische Anlage 1 nicht zwingend ein solcher zur Ermittlung von Füllständen vorgesehen sein, sondern es kann auch ein Sensor 12 zur Ermittlung anderer physikalischer Parameter vorgesehen sein. Beispielsweise könnte auch die Anzahl der Zellen in dem biologischen Medium sensorisch ermittelt werden.

Die bioprozesstechnische Anlage 1 ist vorschlagsgemäß mit einem Sensorpositioniersystem 13 ausgestattet, mit dessen Hilfe der Sensor 12 für einen spezifischen bioprozesstechnischen Anwendungsfall optimal am Container 3 positioniert und dann fixiert werden kann. Das vorschlagsgemäße Sensorpositioniersystem 13 der bioprozesstechnischen Anlage 1 umfasst hier den Container 3 sowie den Halter 5 und den zu fixierenden Sensor 12 der bioprozesstechnischen Anlage 1.

Wesentlich ist nun, dass eine Positionierhilfseinrichtung 14 zur Positionierung des zu fixierenden Sensors 12 in einer vorgegebenen Position am Container 3 vorgesehen ist, dass die Positionierhilfseinrichtung 14 eine unmittelbar am Container 3 oder neben dem Container 3 am Halter 5 angebrachte oder anbringbare Positionierhilfe 15 aufweist, die relativ zu mindestens einem am Container 3 und/oder Halter 5 vorgesehenem Bezugspunkt P in verschiedene Höhen- und/oder Umfangsstellungen bringbar ist, und dass die Positionierhilfseinrichtung 14 ein technisches Speichermedium oder analoges Druckmedium 16 mit einer Zuordnungsliste 17 aufweist, in der mehreren bioprozesstechnischen Anwendungsfällen jeweils eine vorgegebene Höhen- und/oder Umfangsstellung der Positionierhilfe 15 zugeordnet ist.

Die Positionierhilfseinrichtung 14, die ebenfalls Teil des Sensorpositioniersystems 13 ist, wird nun im Folgenden näher erläutert.

Die Anbringung der Positionierhilfe 15 kann wie gesagt unmittelbar am Container 3 erfolgen. Alternativ kann die Anbringung aber auch, und zwar mittelbar oder unmittelbar, am Halter 5 und neben dem Container 3 erfolgen. Die Positionierhilfe 15 kann, wie im Weiteren noch näher erläutert wird, bereits bevor sie in die jeweilige Höhen- und/oder Umfangsstellung gebracht wird, am Container 3 bzw. Halter 5 angebracht sein. Alternativ ist es auch denkbar, zunächst die Positionierhilfe 15 in eine vorgegebene Stellung zu bringen, die im angebrachten Zustand der Positionierhilfe 15 einer bestimmten Höhen- und/oder Umfangsstellung entspricht, und die Positionierhilfe 15 erst anschließend mit dem Container 3 und/oder Halter 5 zu verbinden. Der Sensor 12 ist dann, nachdem sich die Positionierhilfe 15 relativ zu dem Bezugspunkt P in einer einem spezifischen bioprozesstechnischen Anwendungsfall entsprechenden Höhen- und/oder Umfangsstellung befindet, an der Positionierhilfe 15 ausrichtbar und dadurch in der für diesen Anwendungsfall vorgegebenen Position am Container 3 fixierbar. Insbesondere liegt dabei, nachdem sich die Positionierhilfe 15 relativ zum Bezugspunkt P in der dem spezifischen Anwendungsfall entsprechenden Höhen- und/oder Umfangsstellung befindet, eine orthogonal auf die Axialrichtung X gerichtete Projektion der Positionierhilfe 15 innerhalb eines Abschnitts des Containers 13 bzw. der Containerwand.

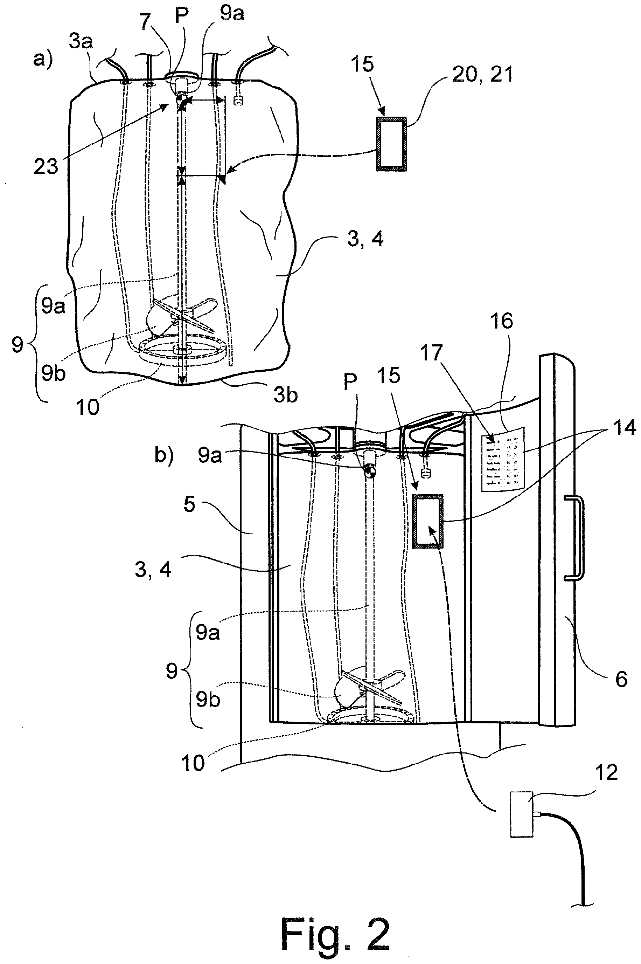

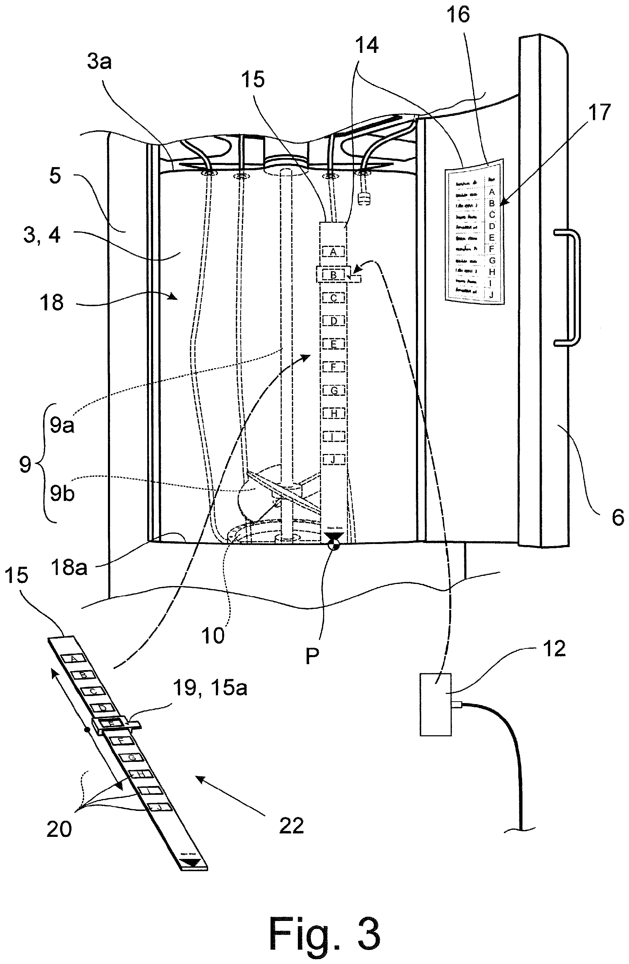

Hier und vorzugsweise handelt es sich, wie in den Fig. 2 und 3 dargestellt, bei dem Speicher- oder Druckmedium 16 um ein solches in Folienform, wobei die Zuordnungsliste 17 aufgedruckt ist. In der Zuordnungsliste 17 sind die Höhenstellungen gemäß Fig. 2 als Abstände zu dem jeweiligen Bezugspunkt P gespeichert. Ferner sind in der Zuordnungsliste 17 auch zusätzliche Umfangsstellungen, hier ebenfalls als Abstände zu dem jeweiligen Bezugspunkt P, gespeichert, die im Weiteren noch näher erläutert werden. Die Liste umfasst also gemäß Fig. 2 mehrere bioprozesstechnische Anwendungsfälle und zu jedem bioprozesstechnischen Anwendungsfall eine Höhenstellung in Form eines Abstands zu dem Bezugspunkt P und eine Umfangsstellung in Form eines Abstands zu dem Bezugspunkt P. Bei dem Ausführungsbeispiel in Fig. 3 sind dagegen in der Zuordnungsliste 17 beispielhaft nur Höhenstellungen als Positionen A, B, ... J, die im angebrachten Zustand der Positionierhilfe 15 relativ zu dem jeweiligen Bezugspunkt P über die Positionierhilfe 15 anzeigbar sind, gespeichert. In letzterem Ausführungsbeispiel weist die Zuordnungsliste 17 zu den einzelnen aufgelisteten bioprozesstechnischen Anwendungsfällen nur jeweils eine Höhenstellung in Form der jeweiligen Position A, B, ... J auf, hier aber keine Umfangsstellungen. Die Umfangsstellung ist immer dieselbe. Aber auch in diesem Ausführungsbeispiel können grundsätzlich auch Umfangsstellungen, beispielsweise ebenfalls in Form entsprechender Positionen, gespeichert sein.

Gemäß einer hier nicht dargestellten Ausführungsform kann auch vorgesehen sein, dass in der Zuordnungsliste 17 den mehreren bioprozesstechnischen Anwendungsfällen jeweils mindestens ein, insbesondere genau ein, am Container 3 und/oder Halter 5 vorgesehener Bezugspunkt P zugeordnet ist, wobei dem jeweiligen Bezugspunkt P jeweils eine vorgegebene Höhen- und/oder Umfangsstellung der Positionierhilfe 15 zugeordnet ist. Verschiedenen bioprozesstechnischen Anwendungsfällen können also unterschiedliche Bezugspunkte P zugeordnet sein. Es ist aber auch denkbar, dass für alle bioprozesstechnischen Anwendungsfälle derselbe Bezugspunkt P vorgesehen ist, wie dies bei den dargestellten Ausführungsbeispielen der Fall ist.

Wie bereits zuvor erwähnt, kann in der Zuordnungsliste 17, wie in Fig. 2 beispielhaft gezeigt, den mehreren bioprozesstechnischen Anwendungsfällen jeweils auch eine vorgegebene Umfangsstellung der Positionierhilfe 15 zugeordnet sein. Zusätzlich kann auch vorgesehen sein, dass in der Zuordnungsliste 17 den mehreren bioprozesstechnischen Anwendungsfällen jeweils mindestens ein, insbesondere genau ein, am Container 3 und/oder Halter 5 vorgesehener Bezugspunkt P zugeordnet ist, wobei dem jeweiligen Bezugspunkt P jeweils eine vorgegebene Umfangsstellung der Positionierhilfe 15 zugeordnet ist. Der Bezugspunkt P, dem die vorgegebene Höhenstellung der Positionierhilfe 15 zugeordnet ist, ist vorzugsweise derselbe Bezugspunkt P, dem die vorgegebene Umfangsstellung der Positionierhilfe 15 zugeordnet ist, wie dies in Fig. 2 der Fall ist. Hier ist also ein gemeinsamer Bezugspunkt P für die Höhenstellung und die Umfangsstellung vorgesehen. Es können aber auch unterschiedliche Bezugspunkte P für die Höhenstellung einerseits und die Umfangsstellung andererseits vorgesehen sein.

Gemäß dem Ausführungsbeispiel in Fig. 2 ist die Positionierhilfe 15 am Container 3 oder Halter 5 als Ganzes zumindest in Axialrichtung X in verschiedenen Positionen relativ zu dem jeweiligen Bezugspunkt P ortsfest anordenbar. "Anordenbar" bedeutet, dass die Positionierhilfe 15 befestigbar, zum Beispiel aufklebbar, aufsteckbar o.dgl. ist, oder an den Container 3 und/oder Halter 5 ansetzbar bzw. darauf aufsetzbar sein kann.

Gemäß Fig. 3 ist die Positionierhilfe 15 alternativ am Container 3 oder Halter 5 ortsfest anbringbar, hier beispielsweise durch Aufsetzen der Positionierhilfe 15 auf eine Kante 18a, hier eine Unterkante, der von der Tür 6 verschließbaren Öffnung 18, wobei vor der Anbringung der Positionierhilfe 15 ein relativ zu dem Container 3 und/oder dem Halter 5 und/oder der Positionierhilfe 15 im Übrigen bewegliches Teil 15a der Positionierhilfe 15, hier ein Schieber 19, entlang der Positionierhilfe 15 im Übrigen in verschiedenen Positionen anordenbar ist. Die Positionen entsprechen jeweils einer bestimmten Position relativ zu dem jeweiligen Bezugspunkt P, wenn sich die Positionierhilfe 15 im angebrachten Zustand befindet. Das bewegliche Teil 15a bzw. der Schieber 19 kann also in eine der in der Zuordnungsliste 17 dem jeweiligen Anwendungsfall zugeordneten Positionen A, B, ... J gebracht werden, wobei diese Positionen jeweils einer Position relativ zu dem jeweiligen Bezugspunkt P entsprechen, wenn sich die Positionierhilfe 15 im angebrachten Zustand befindet.

Der angeordnete Zustand der Positionierhilfe 15 ist in Fig. 3 gestrichelt dargestellt. Es ist nun auch vorstellbar, dass die Positionierhilfe 15 erst am Container 3 oder Halter 5 ortsfest angebracht wird, wobei erst dann ein relativ zum Container 3 und/oder Halter 5 bewegliches Teil 15a der Positionierhilfe 15, insbesondere ein Schieber 19, in Axialrichtung X in die jeweilige Position A, B, ... J relativ zu dem jeweiligen Bezugspunkt P gebracht wird, die dem spezifischen Anwendungsfall entspricht.

Das zuvor Gesagte gilt gleichermaßen auch für die Umfangsstellungen. So ist die Positionierhilfe 15 wie gesagt auch relativ zu mindesten einem am Container 3 und/oder Halter 5 vorgesehenen Bezugspunkt P in verschiedene Umfangsstellungen bringbar. In diesem Fall kann vorgesehen sein, wie dies beispielsweise Fig. 2 zeigt, dass die Positionierhilfe 15 am Container 3 oder Halter 5 als Ganzes in Umfangsrichtung U, also in einer Richtung um die in Axialrichtung X verlaufende Container-Mittelachse M, in verschiedenen Positionen relativ zu dem jeweiligen Bezugspunkt P ortsfest anordenbar ist. Alternativ ist auch hier denkbar, dass die Positionierhilfe 15 am Container 3 oder Halter 5 ortsfest anbringbar ist, wobei vor der Anbringung der Positionierhilfe 15 ein relativ zu dem Container 3 und/oder dem Halter 5 und/oder der Positionierhilfe 15 im Übrigen bewegliches Teil 15a der Positionierhilfe 15, insbesondere ein Schieber 19, quer zur Positionierhilfe 15 im Übrigen in verschiedenen Positionen anordenbar ist, die im angebrachten Zustand der Positionierhilfe 15 jeweils einer bestimmten Position relativ zu dem jeweiligen Bezugspunkt P entsprechen. Wiederum alternativ kann auch vorgesehen sein, dass die Positionierhilfe 15 am Container 3 oder Halter 5 ortsfest angebracht ist, wobei erst dann ein relativ zum Container 3 und/oder Halter 5 bewegliches Teil 15a der Positionierhilfe 15, insbesondere ein Schieber 19, in Umfangsrichtung U in verschiedenen Positionen relativ zu dem jeweiligen Bezugspunkt P anordenbar ist.

Die Positionierhilfe 15 ist hier und vorzugsweise mit dem Container 3 und/oder mit dem Halter 5 jeweils stoffflüssig, formschlüssig und/oder kraftschlüssig verbindbar. Die Verbindung kann eine zerstörungsfrei lösbare Verbindung oder eine dauerhafte, das heißt nicht zerstörungsfrei lösbare Verbindung sein. Vorzugsweise hat die Positionierhilfe 15 im Sensorpositioniersystem 13 selbst keine tragende Funktion, nimmt also keine vom Container 3 und keine vom Halter 5 ausgeübte Last auf. Die Positionierhilfe 15 kann aus einem Kunststoffmaterial, vorzugsweise HDPE (Hart-Polyethylen, High Density Polyethylen), oder aus Metall gebildet sein.

Die Positionierhilfe 15 stellt hier und vorzugsweise mindestens eine Markierung 20 bereit, an der der Sensor 12 ausrichtbar ist. Bei dem Ausführungsbeispiel in Fig. 2 bildet die Positionierhilfe 15 selbst die Markierung 20. Bei dem Ausführungsbeispiel in Fig. 3 weist die Positionierhilfe 15 insbesondere mindestens eine, hier mehrere, Markierungen 20 auf. Markierungen 20 sind insbesondere Striche, Punkte, Schriftzeichen, Wörter, Symbole o.dgl. Der Sensor 12 ist auf diese Weise, nachdem sich die Positionierhilfe 15 relativ zum Bezugspunkt P in einer einem spezifischen bioprozesstechnischen Anwendungsfall entsprechenden Höhen- und/oder Umfangsstellung befindet, an der oder den Markierungen 20 ausrichtbar und dadurch in der für diesen Anwendungsfall vorgegebenen Position am Container 3 fixierbar. Insbesondere liegt dabei, nachdem sich die Positionierhilfe 15 relativ zum Bezugspunkt P in der dem spezifischen Anwendungsfall entsprechenden Höhen- und/oder Umfangsstellung befindet, eine orthogonal auf die Axialrichtung X gerichtete Projektion der Markierung 20 oder Markierungen 20 innerhalb eines Abschnitts des Containers 13 bzw. der Containerwand.

Die Positionierhilfe 15 ist gemäß dem Ausführungsbeispiel in Fig. 2 eine am Container 3, insbesondere unmittelbar am Container 3 oder mit einem geringen Abstand zum Container 3 von maximal 10 cm, vorzugsweise maximal 5 cm, weiter vorzugsweise maximal 1 cm, platzierbare Schablone 21. Eine Schablone ist eine Vorlage, die Begrenzungen bildet, innerhalb derer der Sensor 12 platzierbar ist. In Fig. 2 hat die Schablone 21 beispielhaft die Form eines vollständig umlaufenden Rahmens. Die Schablone 21 ist insbesondere aufklebbar und wird hier und vorzugsweise, wie in Fig. 2 dargestellt, unmittelbar auf den Container 3 aufgeklebt.

In einer Variante ist die Positionierhilfe 15, wie im Ausführungsbeispiel in Fig. 3 gezeigt, ein am Container 3 oder neben dem Container 3 am Halter 5 platzierbarer, mehrere Markierungen 20 aufweisender Markierungsträger 22. Die mehreren Markierungen 20 sind beispielsweise in Form einer Teilung (Skala) angeordnet, das heißt in Form einer Mehrzahl gleichmäßig oder ungleichmäßig beabstandeter Markierungen 20, insbesondere Striche, Punkte o.dgl. Der Markierungsträger 22 ist vorzugsweise als Stab oder Band ausgestaltet und insbesondere am Container 3 und/oder Halter 5 befestigbar oder an den Container 3 und/oder Halter 5 ansetzbar oder darauf aufsetzbar. Weiter vorzugsweise ist am Markierungsträger 22 wie in Fig. 3 mindestens ein, hier genau ein, zwischen den Markierungen 20 bewegbarer Schieber 19 angeordnet.

In einer anderen Variante, die hier nicht dargestellt ist, sind als Positionierhilfe 15 mehrere Markierungen 20 unmittelbar auf dem Container 3 und/oder Halter 5 aufgebracht, insbesondere aufgedruckt und/oder aufgeklebt. Die mehreren Markierungen 20 sind beispielsweise in Form einer Teilung (Skala) angeordnet.

Die Positionierhilfe 15 und die Zuordnungsliste 17 können auf verschiedene Weise relativ zum Sensorpositioniersystem13 im Übrigen angeordnet werden.

So ist die Positionierhilfe 15 vorzugsweise ein von dem Halter 5 und/oder der Tür 6 separates Teil. "Separat" bedeutet, dass die Positionierhilfe 15 im nicht angebrachten Zustand von dem Halter 5 und/oder der Tür 6 getrennt ist. Die Positionierhilfe 15 kann aber grundsätzlich auch in den Halter 5 und/oder die Tür 6 integriert sein, beispielsweise als in den Halter 5 und/oder in die Tür 6 integrierte Schablone.

Zusätzlich oder alternativ ist das Speicher- oder Druckmedium 16 von dem Container 3 und/oder Halter 5 getrennt, also nicht mit dem Container 3 und/oder Halter 5 verbunden, oder am Container 3 und/oder Halter 5 angebracht, insbesondere angeklebt, beispielsweise wie hier an der Innen- oder Außenseite der Tür 6.

Ein oder mehrere Bezugspunkte P am Container 3, relativ zu dem die Positionierhilfe 15 in verschiedene Höhen- und/oder Umfangsstellungen bringbar ist, werden insbesondere von dem oberen oder unteren Containerende 3a, 3b, von einer Beutelnaht, von einem mechanischen Absatz, beispielsweise einem Vorsprung oder einer Nut, am Container 3, von einem Anschlussstück 7, beispielsweise einem Sensor-Anschlussstück, Probenahme-Anschlussstück etc., und/oder wie in Fig. 2 von einem mechanischen Absatz 23, beispielsweise einem Vorsprung oder einer Nut, an der Rührwelle 9a des Rührwerks 9 gebildet. Ein oder mehrere Bezugspunkte P am Halter 5, relativ zu dem die Positionierhilfe 15 in verschiedene Höhen- und/oder Umfangsstellungen bringbar ist, werden insbesondere von einer Kante 18a, beispielsweise Ober-, Unter- oder Seitenkante, insbesondere einer Öffnung 18, und/oder von einem mechanischen Absatz, beispielsweise einem Vorsprung oder einer Nut, am Halter 5 gebildet.

Gemäß einer weiteren, hier nicht dargestellten Lehre, der eigenständige Bedeutung zukommt, wird ein Sensorpositioniersystem 13 für eine bioprozesstechnische Anlage 1 beansprucht, wobei ein Container 3, insbesondere Einwegcontainer, zur Aufnahme eines biologischen Mediums vorgesehen ist, der sich im installierten Zustand in Axialrichtung X von einem oberen Containerende 3a zu einem unteren Containerende 3b erstreckt, wobei ein Halter 5 zum Halten des Containers 3 im installierten Zustand vorgesehen ist, wobei ein an dem Container 3 zu fixierender Sensor 12 vorgesehen ist. Weiter ist eine Positionierhilfseinrichtung 14 zur Positionierung des zu fixierenden Sensors 12 in einer vorgegebenen Position am Container 3 vorgesehen, wobei die Positionierhilfseinrichtung 14 eine unmittelbar am Container 3 oder neben dem Container 3 am Halter 5 angebrachte Positionierhilfe 15 aufweist, relativ zu der der Sensor 12 in verschiedene Höhen- und/oder Umfangsstellungen bringbar ist, und wobei die Positionierhilfseinrichtung 14 ein technisches Speichermedium oder analoges Druckmedium 16 mit einer Zuordnungsliste 17 aufweist, in der mehreren bioprozesstechnischen Anwendungsfällen jeweils eine vorgegebene Höhen- und/oder Umfangsstellung des Sensors 12 zugeordnet ist.

Wesentlich ist hierbei, dass der Sensor 12 relativ zu einer insbesondere festen, das heißt unbeweglichen, Positionierhilfe 15, in eine bestimmte Höhen- und/oder Umfangsstellung gebracht und dann in der jeweiligen Position relativ zu der Positionierhilfe 15 am Container 3 fixiert wird. Als Positionierhilfe 15 sind vorzugsweise mehrere Markierungen 20 unmittelbar auf dem Container 3 und/oder Halter 5 aufgebracht, insbesondere aufgedruckt und/oder aufgeklebt. Die mehreren Markierungen 20 sind beispielsweise in Form einer Teilung (Skala), insbesondere Maßteilung, angeordnet. Das Sensorpositioniersystem 13 und/oder die bioprozesstechnische Anlage 1 kann auch einzelne der Merkmale aufweisen, die in Hinblick auf das Sensorpositioniersystem 13 gemäß der ersten Lehre beschrieben worden sind. Es darf hinsichtlich des vorschlagsgemäßen Sensorpositioniersystems 13 der weiteren Lehre auf alle Ausführungen zu der erstgenannten Lehre verwiesen werden.

Gemäß einer weiteren Lehre, der ebenfalls eigenständige Bedeutung zukommt, wird eine bioprozesstechnische Anlage 1, insbesondere ein Bioreaktor 2 oder ein Prozess-Mischsystem, mit einem vorschlagsgemäßen Sensorpositioniersystem 13 beansprucht. Es darf hinsichtlich der vorschlagsgemäßen bioprozesstechnischen Anlage 1 auf alle Ausführungen zu der erstgenannten und zweitgenannten Lehre verwiesen werden.

Gemäß einer weiteren Lehre, der ebenfalls eigenständige Bedeutung zukommt, wird ein Verfahren zur Positionierung eines Sensors 12 einer bioprozesstechnische Anlage 1, insbesondere unter Verwendung eines vorschlagsgemäßen Sensorpositioniersystems 13, beansprucht, wobei ein Container 3, insbesondere Einwegcontainer, zur Aufnahme eines biologischen Mediums bereitgestellt wird, wobei der Container 3 von einem Halter 5 aufgenommen wird, der den Container 3 hält, derart, dass sich der Container 3 in Axialrichtung X von einem oberen Containerende 3a zu einem unteren Containerende 3b erstreckt. Dabei ist vorgesehen, dass nach der Aufnahme des Containers 3 durch den Halter 5 der Sensor 12 an einer Positionierhilfe 15, die fest oder beweglich sein kann, einer Positionierhilfseinrichtung 14 ausgerichtet wird und am Container 3 fixiert wird, wobei die Ausrichtung basierend auf einer vorgegebenen Höhenstellung und/oder basierend auf einer vorgegebenen Umfangsstellung erfolgt, die in einer Zuordnungsliste 17 eines technischen Speichermediums oder analogen Druckmediums 16 einem von mehreren bioprozesstechnischen Anwendungsfällen zugeordnet ist. Es darf hinsichtlich des vorschlagsgemäßen Verfahrens auf alle Ausführungen zu den weiteren Lehren verwiesen werden.

Wie in Fig. 2 dargestellt ist es vorzugsweise so, dass die Positionierhilfe 15 nach der Aufnahme des Containers 3 durch den Halter 5 unmittelbar am Container 3 oder Halter 5 angebracht wird. Die Positionierhilfe 15 wird dabei im Zuge ihrer Anbringung relativ zu mindestens einem am Container 3 und/oder Halter 5 vorgesehenen Bezugspunkt P in eine vorgegebene Höhenstellung und in eine vorgegebene Umfangsstellung gebracht (Fig. 2a). Der Sensor 12 wird dann an der Positionierhilfe 15 ausgerichtet (Fig. 2b). Die vorgegebene Höhenstellung bzw. Umfangsstellung, basierend auf der die Ausrichtung des Sensors 12 erfolgt, ist also eine Höhenstellung bzw. Umfangsstellung der Positionierhilfe 15, wobei die Positionierhilfe 15 im Zuge ihrer Anbringung in die vorgegebene Höhenstellung bzw. Umfangsstellung gebracht wird. Insbesondere wird die Positionierhilfe 15 dadurch in ihre vorgegebene Höhenstellung bzw. Umfangsstellung gebracht, dass sie, im Zuge ihrer Anbringung, als Ganzes in einer vorgegebenen Position relativ zu dem jeweiligen Bezugspunkt P angeordnet wird. Dann wird der Sensor 12, nachdem sich die Positionierhilfe 15 relativ zum Bezugspunkt P in der dem spezifischen bioprozesstechnischen Anwendungsfall entsprechenden Höhenstellung und/oder Umfangsstellung befindet, an der Positionierhilfe 15 ausgerichtet und am Container 3 fixiert.

Zusätzlich oder alternativ kann auch vorgesehen sein, dass die Positionierhilfe 15 bereits vor dem Einstellen der vorgegebenen Höhenstellung und/oder vor dem Einstellen der vorgegebenen Umfangsstellung, gegebenenfalls auch bereits vor der Aufnahme des Containers 3 durch den Halter 5, unmittelbar am Container 3 oder Halter 5 angebracht ist. Die Positionierhilfe 15 wird dann relativ zu mindestens einem am Container 3 und/oder Halter 5 vorgesehenen Bezugspunkt P in die vorgegebene Höhenstellung bzw. Umfangsstellung gebracht, wobei der Sensor 12 danach an der Positionierhilfe 15 ausgerichtet wird. Auch hierbei ist die vorgegebene Höhenstellung bzw. Umfangsstellung, basierend auf der die Ausrichtung des Sensors 12 erfolgt, eine Höhenstellung bzw. Umfangsstellung der Positionierhilfe 15, wobei die Positionierhilfe 15 aber schon angebracht ist, bevor sie in die vorgegebene Höhenstellung bzw. Umfangsstellung gebracht wird. Insbesondere wird die Positionierhilfe 15 dadurch in ihre vorgegebene Höhenstellung bzw. Umfangsstellung gebracht, dass ein bewegliches Teil 15a der Positionierhilfe 15, insbesondere ein Schieber 19, in einer vorgegebenen Position relativ zu dem jeweiligen Bezugspunkt P angeordnet wird. Dann wird der Sensor 12, nachdem sich die Positionierhilfe 15 relativ zum Bezugspunkt P in der dem spezifischen bioprozesstechnischen Anwendungsfall entsprechenden Höhenstellung und/oder Umfangsstellung befindet, an der Positionierhilfe 15 ausgerichtet.

Bei dem Ausführungsbeispiel in Fig. 3 ist es so, dass die Positionierhilfe 15 vor der Anbringung am Container 3 oder Halter 5, insbesondere nach der Aufnahme des Containers 3 durch den Halter 5, in eine vorgegebene Stellung gebracht wird, die im angebrachten Zustand der Positionierhilfe 15 jeweils einer bestimmten Höhenstellung und/oder einer vorgegebenen Umfangsstellung relativ zu mindestens einem am Container 3 und/oder Halter 5 vorgesehenen Bezugspunkt P entspricht. Die Positionierhilfe 15 wird dabei in die vorgegebene Stellung gebracht, indem das bewegliche Teil 15a bzw. hier der Schieber 19 relativ zur Positionierhilfe 15 im Übrigen verstellt wird. Danach wird die Positionierhilfe unmittelbar am Container 3 oder Halter 5 angebracht. Der Sensor 12 wird dann an der Positionierhilfe 15 ausgerichtet. Auch hierbei ist die vorgegebene Höhenstellung und/oder Umfangsstellung, basierend auf der die Ausrichtung des Sensors 12 erfolgt, eine Höhenstellung bzw. Umfangsstellung der Positionierhilfe 15, wobei die Positionierhilfe 15 aber erst angebracht wird, nachdem sie in eine der vorgegebenen Höhenstellung bzw. Umfangsstellung entsprechende Stellung gebracht worden ist. Insbesondere wird auch hier die Positionierhilfe 15 dadurch in ihre vorgegebene Höhenstellung bzw. Umfangsstellung gebracht, dass ein bewegliches Teil 15a der Positionierhilfe 15, beispielsweise ein Schieber 19, in einer vorgegebenen Position relative zur Positionierhilfe 15 im Übrigen angeordnet wird, welche Position im angebrachten Zustand der Positionierhilfe 15 der vorgegebenen Position relativ zu dem jeweiligen Bezugspunkt P entspricht. Dann wird der Sensor 12, nachdem die Positionierhilfe 15 angebracht worden ist, an der Positionierhilfe 15 ausgerichtet.

Gemäß einem alternativen und nicht in den Ausführungsbeispielen dargestellten Verfahren kann auch vorgesehen sein, dass die Positionierhilfe 15 bereits vor der Aufnahme des Containers 3 durch den Halter 5 unmittelbar am Container 3 oder Halter 5 angebracht ist. Der Sensor 12 wird dann nach der Aufnahme des Containers 3 durch den Halter 5 relativ zu der Positionierhilfe 15 in eine vorgegebene Höhenstellung und/oder in eine vorgegebenen Umfangsstellung gebracht und anschließend an der Positionierhilfe 15 ausgerichtet. Hierbei ist die vorgegebene Höhenstellung und/oder Umfangsstellung, basierend auf der die Ausrichtung des Sensors 12 erfolgt, eine Höhenstellung bzw. Umfangsstellung des Sensors 12, wobei die Positionierhilfe 15 insbesondere fest, das heißt unbeweglich, am Container 3 bzw. Halter 5 angebracht ist. Der Sensor 12 wird also relativ zu einer insbesondere festen, das heißt unbeweglichen, Positionierhilfe 15, in eine bestimmte Höhenstellung bzw. Umfangsstellung gebracht und dann in der jeweiligen Position relativ zu der Positionierhilfe 15 am Container 3 fixiert.

## Patentansprüche

1. Sensorpositioniersystem für eine bioprozesstechnische Anlage (1),
wobei ein Container (3), insbesondere Einwegcontainer, zur Aufnahme eines biologischen Mediums vorgesehen ist, der sich im installierten Zustand in Axialrichtung (X) von einem oberen Containerende (3a) zu einem unteren Containerende (3b) erstreckt,
wobei ein Halter (5) zum Halten des Containers (3) im installierten Zustand vorgesehen ist,
wobei ein an dem Container (3) zu fixierender Sensor (12) vorgesehen ist,
**dadurch gekennzeichnet,**
**dass** eine Positionierhilfseinrichtung (14) zur Positionierung des zu fixierenden Sensors (12) in einer vorgegebenen Position am Container (3) vorgesehen ist, dass die Positionierhilfseinrichtung (14) eine unmittelbar am Container (3) oder neben dem Container (3) am Halter (5) angebrachte oder anbringbare Positionierhilfe (15) aufweist, die relativ zu mindestens einem am Container (3) und/oder Halter (5) vorgesehenen Bezugspunkt (P) in verschiedene Höhen- und/oder Umfangsstellungen bringbar ist, und
**dass** die Positionierhilfseinrichtung (14) ein technisches Speichermedium oder analoges Druckmedium (16) mit einer Zuordnungsliste (17) aufweist, in der mehreren bioprozesstechnischen Anwendungsfällen jeweils eine vorgegebene Höhen- und/oder Umfangsstellung der Positionierhilfe (15) zugeordnet ist.

2. Sensorpositioniersystem nach Anspruch 1, **dadurch gekennzeichnet, dass** das Speicher- oder Druckmedium (16) ein elektronisches, magnetisches, optisches oder fotografisches Speichermedium, oder ein Druckmedium in Papierform oder Folienform umfasst.

3. Sensorpositioniersystem nach Anspruch 1 oder 2, **dadurch gekennzeichnet, dass** in der Zuordnungsliste (17) den mehreren bioprozesstechnischen Anwendungsfällen jeweils mindestens ein, insbesondere genau ein, am Container (3) und/oder Halter (5) vorgesehener Bezugspunkt (P) zugeordnet ist, wobei dem jeweiligen Bezugspunkt (P) jeweils eine vorgegebene Höhen- und/oder Umfangsstellung der Positionierhilfe (15) zugeordnet ist.

4. Sensorpositioniersystem nach einem der vorhergehenden Ansprüche, **dadurch gekennzeichnet, dass** die Positionierhilfe (15) am Container (3) oder Halter (5) als Ganzes in Axialrichtung (X) in verschiedenen Positionen relativ zu dem jeweiligen Bezugspunkt (P) ortsfest anordenbar ist, oder, dass die Positionierhilfe (15) am Container (3) oder Halter (5) ortsfest anbringbar ist, wobei vor der Anbringung der Positionierhilfe (15) ein relativ zu dem Container (3) und/oder dem Halter (5) und/oder der Positionierhilfe (15) im Übrigen bewegliches Teil (15a) der Positionierhilfe (15) entlang der Positionierhilfe (15) im Übrigen in verschiedenen Positionen anordenbar ist, die im angebrachten Zustand der Positionierhilfe (15) jeweils einer bestimmten Position relativ zu dem jeweiligen Bezugspunkt (P) entsprechen, oder, dass die Positionierhilfe (15) am Container (3) oder Halter (5) ortsfest angebracht ist, wobei ein relativ zum Container (3) und/oder Halter (5) bewegliches Teil (15a) der Positionierhilfe (15) in Axialrichtung (X) in verschiedenen Positionen relativ zu dem jeweiligen Bezugspunkt (P) anordenbar ist.

5. Sensorpositioniersystem nach einem der vorhergehenden Ansprüche, **dadurch gekennzeichnet, dass** die Positionierhilfe (15) am Container (3) oder Halter (5) als Ganzes in Umfangsrichtung (U) in verschiedenen Positionen relativ zu dem jeweiligen Bezugspunkt (P) ortsfest anordenbar ist, oder, dass die Positionierhilfe (15) am Container (3) oder Halter (5) ortsfest anbringbar ist, wobei vor der Anbringung der Positionierhilfe (15) ein relativ zu dem Container (3) und/oder dem Halter (5) und/oder der Positionierhilfe (15) im Übrigen bewegliches Teil (15a) der Positionierhilfe (15) quer zur Positionierhilfe (15) im Übrigen in verschiedenen Positionen anordenbar ist, die im angebrachten Zustand der Positionierhilfe (15) jeweils einer bestimmten Position relativ zu dem jeweiligen Bezugspunkt (P) entsprechen, oder, dass die Positionierhilfe (15) am Container (3) oder Halter (5) ortsfest angebracht ist, wobei ein relativ zum Container (3) und/oder Halter (5) bewegliches Teil (15a) der Positionierhilfe (15) in Umfangsrichtung (U) in verschiedenen Positionen relativ zu dem jeweiligen Bezugspunkt (P) anordenbar ist.

6. Sensorpositioniersystem nach einem der vorhergehenden Ansprüche, **dadurch gekennzeichnet, dass** die Positionierhilfe (15) mit dem Container (3) und/oder mit dem Halter (5) stoffschlüssig, formschlüssig und/oder kraftschlüssig verbindbar ist.

7. Sensorpositioniersystem nach einem der vorhergehenden Ansprüche, **dadurch gekennzeichnet, dass** die Positionierhilfe (15) mindestens eine Markierung (20) bereitstellt, an der der Sensor (12) ausrichtbar ist, vorzugsweise, dass die Positionierhilfe (15) die mindestens eine Markierung (20) aufweist oder selbst bildet.

8. Sensorpositioniersystem nach einem der vorhergehenden Ansprüche, **dadurch gekennzeichnet, dass** die Positionierhilfe (15) eine am Container (3) platzierbare Schablone (21) ist, die insbesondere auf den Container (3) aufklebbar ist, oder, dass die Positionierhilfe (15) ein am Container (3) oder neben dem Container (3) am Halter (5) platzierbarer, mehrere Markierungen (20), insbesondere in Form einer Teilung, aufweisender Markierungsträger (22), vorzugsweise ein als Stab oder Band ausgestalteter Markierungsträger (22), ist, der insbesondere am Container (3) und/oder Halter (5) befestigbar oder an dem Container (3) und/oder Halter (5) ansetzbar ist, vorzugsweise, dass am Markierungsträger (22) mindestens ein zwischen den Markierungen (20) bewegbarerer Schieber (19) angeordnet ist, oder, dass als Positionierhilfe (15) mehrere unmittelbar auf dem Container (3) und/oder Halter (5) aufgebrachte Markierungen (20), insbesondere in Form einer Teilung, vorgesehen sind.

9. Sensorpositioniersystem nach einem der vorhergehenden Ansprüche, **dadurch gekennzeichnet, dass** die Positionierhilfe (15) separat von dem Halter (5) und/oder einer Tür (6) des Halters (5) ist oder in den Halter (5) und/oder eine Tür (6) des Halters (5) integriert ist, und/oder, dass das Speicher- oder Druckmedium (16) von dem Container (3) und/oder Halter (5) getrennt ist oder am Container (3) und/oder Halter (5) angebracht, insbesondere angeklebt, ist.

10. Sensorpositioniersystem nach einem der vorhergehenden Ansprüche, **dadurch gekennzeichnet, dass** der Container (3) ein Bioprozessbeutel (4) oder ein formstabiles Gefäß, vorzugsweise für einen Bioreaktor (2) oder einen Prozess-Mischsystem, ist, und/oder, dass der Container (3) ein integriertes Rührwerk (9) aufweist.

11. Sensorpositioniersystem nach einem der vorhergehenden Ansprüche, **dadurch gekennzeichnet, dass** ein oder mehrere Bezugspunkte (P) am Container (3) von dem oberen oder dem unteren Containerende (3a, 3b), von einer Beutelnaht, von einem mechanischen Absatz am Container (3), von einem mechanischen Absatz (23) an einer Rührwelle (9a) des Rührwerks (9) und/oder von einem Anschlussstück (7) am Container (3) gebildet werden, und/oder, dass ein oder mehrere Bezugspunkte (P) am Halter (5) von einer Kante (18a), insbesondere einer Öffnung (18), und/oder von einem mechanischen Absatz am Halter (5) gebildet werden.

12. Sensorpositioniersystem nach einem der vorhergehenden Ansprüche, **dadurch gekennzeichnet, dass** der Sensor (12) als induktiver Sensor, als kapazitiver Sensor, als optischer Sensor, als Ultraschallsensor, als Mikrowellensensor, als Radarsensor oder als Sensor zur Messung der elektrischen Leitfähigkeit oder Wärmeleitfähigkeit ausgestaltet ist, und/oder, dass der Sensor (12) als Füllstandsensor, vorzugsweise als Füllstandgrenzschalter oder als Sensor zur kontinuierlichen Füllstandmessung, ausgestaltet ist und insbesondere ein Schaumsensor ist.

13. Sensorpositioniersystem für eine bioprozesstechnische Anlage (1),
wobei ein Container (3), insbesondere Einwegcontainer, zur Aufnahme eines biologischen Mediums vorgesehen ist, der sich im installierten Zustand in Axialrichtung (X) von einem oberen Containerende (3a) zu einem unteren Containerende (3b) erstreckt,
wobei ein Halter (5) zum Halten des Containers (3) im installierten Zustand vorgesehen ist,
wobei ein an dem Container (3) zu fixierender Sensor (12) vorgesehen ist,
**dadurch gekennzeichnet,**
**dass** eine Positionierhilfseinrichtung (14) zur Positionierung des zu fixierenden Sensors (12) in einer vorgegebenen Position am Container (3) vorgesehen ist, dass die Positionierhilfseinrichtung (14) eine unmittelbar am Container (3) oder neben dem Container (3) am Halter (5) angebrachte Positionierhilfe (15) aufweist, relativ zu der der Sensor (12) in verschiedene Höhen- und/oder Umfangsstellungen bringbar ist, und
**dass** die Positionierhilfseinrichtung (14) ein technisches Speichermedium oder analoges Druckmedium (16) mit einer Zuordnungsliste (17) aufweist, in der mehreren bioprozesstechnischen Anwendungsfällen jeweils eine vorgegebene Höhen- und/oder Umfangsstellung des Sensors (12) zugeordnet ist.

14. Bioprozesstechnische Anlage, insbesondere Bioreaktor (2) oder Prozess-Mischsystem, mit einem Sensorpositioniersystem (13) nach einem der vorhergehenden Ansprüche.

15. Verfahren zur Positionierung eines Sensors (12) einer bioprozesstechnischen Anlage (1), insbesondere unter Verwendung eines Sensorpositioniersystems (13) nach einem der Ansprüche 1 bis 14,
wobei ein Container (3), insbesondere Einwegcontainer, zur Aufnahme eines biologischen Mediums bereitgestellt wird,
wobei der Container (3) von einem Halter (5) aufgenommen wird, der den Container (3) hält, derart, dass sich der Container (3) in Axialrichtung (X) von einem oberen Containerende (3a) zu einem unteren Containerende (3b) erstreckt,
**dadurch gekennzeichnet,**
**dass** nach der Aufnahme des Containers (3) durch den Halter (5) der Sensor (12) an einer Positionierhilfe (15) einer Positionierhilfseinrichtung (14) ausgerichtet wird und am Container (3) fixiert wird, wobei die Ausrichtung basierend auf einer vorgegebenen Höhen- und/oder Umfangsstellung erfolgt, die in einer Zuordnungsliste (17) eines technischen Speichermediums oder analogen Druckmediums (16) einem von mehreren bioprozesstechnischen Anwendungsfällen zugeordnet ist.

16. Verfahren nach Anspruch 15, **dadurch gekennzeichnet, dass** die Positionierhilfe (15) nach der Aufnahme des Containers (3) durch den Halter (5) unmittelbar am Container (3) oder Halter (5) angebracht wird, wobei die Positionierhilfe (15) im Zuge ihrer Anbringung relativ zu mindestens einem am Container (3) und/oder Halter (5) vorgesehenen Bezugspunkt (P) in die vorgegebene Höhenstellung und/oder Umfangsstellung gebracht wird, wobei der Sensor (12) dann an der Positionierhilfe (15) ausgerichtet wird, oder, dass die Positionierhilfe (15) bereits vor dem Einstellen der vorgegebenen Höhenstellung und/oder Umfangsstellung, gegebenenfalls auch bereits vor der Aufnahme des Containers (3) durch den Halter (5), unmittelbar am Container (3) oder Halter (5) angebracht ist, wobei die Positionierhilfe (15) dann relativ zu mindestens einem am Container (3) und/oder Halter (5) vorgesehenen Bezugspunkt (P) in die vorgegebene Höhenstellung und/oder Umfangsstellung, gebracht wird, wobei der Sensor (12) dann an der Positionierhilfe (15) ausgerichtet wird, oder, dass die Positionierhilfe (15) vor der Anbringung am Container (3) oder Halter (5), insbesondere nach der Aufnahme des Containers (3) durch den Halter (5), in eine vorgegebene Stellung gebracht wird, die im angebrachten Zustand der Positionierhilfe (15) jeweils einer bestimmten Höhenstellung und/oder Umfangsstellung, relativ zu mindestens einem am Container (3) und/oder Halter (5) vorgesehenen Bezugspunkt (P) entspricht, wobei die Positionierhilfe (15) dann unmittelbar am Container (3) oder Halter (5) angebracht wird, wobei der Sensor (12) dann an der Positionierhilfe (15) ausgerichtet wird.

17. Verfahren nach Anspruch 15, **dadurch gekennzeichnet, dass** die Positionierhilfe (15) bereits vor der Aufnahme des Containers (3) durch den Halter (5) unmittelbar am Container (3) oder Halter (5) angebracht ist, wobei der Sensor (12) nach der Aufnahme des Containers (3) durch den Halter (5) relativ zu der Positionierhilfe (15) in die vorgegebene Höhenstellung und/oder Umfangsstellung, gebracht wird und an der Positionierhilfe (15) ausgerichtet wird.

## Claims

1. Sensor positioning system for a bioprocess engineering unit (1),
wherein a container (3), in particular a single-use container, is provided for receiving a biological medium, which container in the installed state extends in an axial direction (X) from an upper container end (3a) to a lower container end (3b),
wherein a holder (5) is provided for holding the container (3) in the installed state,
wherein a sensor (12) is provided, which is to be fixed to the container (3),
**characterized**
**in that** a positioning aid device (14) is provided for positioning the sensor (12) to be fixed in a predefined position on the container (3),
**in that** the positioning aid device (14) comprises a positioning aid (15), which is or can be mounted directly on the container (3) or next to the container (3) on the holder (5) and can be brought into different height positions and/or circumferential positions relative to at least one reference point (P) provided on the container (3) and/or holder (5), and
**in that** the positioning aid device (14) comprises a technical storage medium or analogue print medium (16) with an assignment list (17), in which a predefined height position and/or circumferential position of the positioning aid (15) is assigned to each of a plurality of bioprocess engineering applications.

2. Sensor positioning system according to claim 1, **characterized in that** the storage medium or print medium (16) comprises an electronic, magnetic, optical or photographic storage medium, or a print medium in paper or film form.

3. Sensor positioning system according to claim 1 or 2, **characterized in that**, in the assignment list (17), at least one, in particular precisely one, reference point (P) provided on the container (3) and/or holder (5) is assigned to each of the plurality of bioprocess engineering applications, wherein in each case a predefined height position and/or circumferential position of the positioning aid (15) is assigned to the respective reference point (P).

4. Sensor positioning system according to any one of the preceding claims, **characterized in that** the positioning aid (15) can be arranged in a stationary manner on the container (3) or holder (5) as a whole in the axial direction (X) in different positions relative to the respective reference point (P), or **in that** the positioning aid (15) can be mounted in a stationary manner on the container (3) or holder (5), wherein, prior to mounting the positioning aid (15), a portion (15a) of the positioning aid (15) that is movable relative to the container (3) and/or the holder (5) and/or the rest of the positioning aid (15) can be arranged along the rest of the positioning aid (15) in different positions which, in the mounted state of the positioning aid (15), correspond in each case to a specific position relative to the respective reference point (P), or **in that** the positioning aid (15) is mounted in a stationary manner on the container (3) or holder (5), wherein a portion (15a) of the positioning aid (15) that is movable relative to the container (3) and/or holder (5) can be arranged in the axial direction (X) in different positions relative to the respective reference point (P).

5. Sensor positioning system according to any one of the preceding claims, **characterized in that** the positioning aid (15) can be arranged in a stationary manner on the container (3) or holder (5) as a whole in the circumferential direction (U) in different positions relative to the respective reference point (P), or **in that** the positioning aid (15) can be mounted in a stationary manner on the container (3) or holder (5), wherein, prior to mounting the positioning aid (15), a portion (15a) of the positioning aid (15) that is movable relative to the container (3) and/or the holder (5) and/or the rest of the positioning aid (15) can be arranged transversely to the rest of the positioning aid (15) in different positions which, in the mounted state of the positioning aid (15), correspond in each case to a specific position relative to the respective reference point (P), or **in that** the positioning aid (15) is mounted in a stationary manner on the container (3) or holder (5), wherein a portion (15a) of the positioning aid (15) that is movable relative to the container (3) and/or holder (5) can be arranged in the circumferential direction (U) in different positions relative to the respective reference point (P).

6. Sensor positioning system according to any one of the preceding claims, **characterized in that** the positioning aid (15) can be connected to the container (3) and/or to the holder (5) in a materially bonded, form-fitting and/or force-fitting manner.

7. Sensor positioning system according to any one of the preceding claims, **characterized in that** the positioning aid (15) provides at least one marking (20), with which the sensor (12) can be aligned, preferably **in that** the positioning aid (15) has or itself forms the at least one marking (20).

8. Sensor positioning system according to any one of the preceding claims, **characterized in that** the positioning aid (15) is a template (21), which can be placed on the container (3) and in particular can be adhesively bonded to the container (3), or in that the positioning aid (15) is a marking carrier (22), preferably a marking carrier (22) designed as a rod or tape, which can be placed on the container (3) or next to the container (3) on the holder (5) and has a plurality of markings (20), in particular in the form of a scale, and which in particular can be fastened to the container (3) and/or holder (5) or can be placed on the container (3) and/or holder (5), preferably **in that** at least one slide (19) is arranged on the marking carrier (22) and is movable between the markings (20), or **in that** a plurality of markings (20) applied directly to the container (3) and/or holder (5), in particular in the form of a scale, are provided as the positioning aid (15).

9. Sensor positioning system according to any one of the preceding claims, **characterized in that** the positioning aid (15) is separate from the holder (5) and/or from a door (6) of the holder (5) or is integrated in the holder (5) and/or in a door (6) of the holder (5), and/or **in that** the storage medium or print medium (16) is separate from the container (3) and/or holder (5) or is mounted on the container (3) and/or holder (5), in particular by adhesive bonding.

10. Sensor positioning system according to any one of the preceding claims, **characterized in that** the container (3) is a bioprocess bag (4) or a dimensionally stable vessel, preferably for a bioreactor (2) or a process mixing system, and/or **in that** the container (3) has an integrated stirring apparatus (9).

11. Sensor positioning system according to any one of the preceding claims, **characterized in that** one or more reference points (P) on the container (3) are formed by the upper or the lower container end (3a, 3b), by a bag seam, by a mechanical stop on the container (3), by a mechanical stop (23) on a stirring shaft (9a) of the stirring apparatus (9) and/or by a connection piece (7) on the container (3), and/or **in that** one or more reference points (P) on the holder (5) are formed by an edge (18a), in particular of an opening (18), and/or by a mechanical stop on the holder (5).

12. Sensor positioning system according to any one of the preceding claims, **characterized in that** the sensor (12) is designed as an inductive sensor, as a capacitive sensor, as an optical sensor, as an ultrasound sensor, as a microwave sensor, as a radar sensor or as a sensor for measuring the electrical conductivity or thermal conductivity, and/or **in that** the sensor (12) is designed as a fill level sensor, preferably as a fill level limit switch or as a sensor for continuously measuring the fill level, and in particular is a foam sensor.

13. Sensor positioning system for a bioprocess engineering unit (1),
wherein a container (3), in particular a single-use container, is provided for receiving a biological medium, which container in the installed state extends in an axial direction (X) from an upper container end (3a) to a lower container end (3b),
wherein a holder (5) is provided for holding the container (3) in the installed state,
wherein a sensor (12) is provided, which is to be fixed to the container (3),
**characterized**
**in that** a positioning aid device (14) is provided for positioning the sensor (12) to be fixed in a predefined position on the container (3),
**in that** the positioning aid device (14) comprises a positioning aid (15), which is mounted directly on the container (3) or next to the container (3) on the holder (5) and relative to which the sensor (12) can be brought into different height positions and/or circumferential positions, and
**in that** the positioning aid device (14) comprises a technical storage medium or analogue print medium (16) with an assignment list (17), in which a predefined height position and/or circumferential position of the sensor (12) is assigned to each of a plurality of bioprocess engineering applications.

14. Bioprocess engineering unit, in particular a bioreactor (2) or a process mixing system, having a sensor positioning system (13) according to any one of the preceding claims.

15. Method for positioning a sensor (12) of a bioprocess engineering unit (1), in particular using a sensor positioning system (13) according to any one of claims 1 to 14,
wherein a container (3), in particular a single-use container, is provided for receiving a biological medium,
wherein the container (3) is received by a holder (5), which holds the container (3) in such a way that the container (3) extends in an axial direction (X) from an upper container end (3a) to a lower container end (3b),
**characterized**
**in that**, once the container (3) has been received by the holder (5), the sensor (12) is aligned with a positioning aid (15) of a positioning aid device (14) and is fixed to the container (3), wherein the alignment takes place on the basis of a predefined height position and/or circumferential position which is assigned to one of a plurality of bioprocess engineering applications in an assignment list (17) of a technical storage medium or analogue print medium (16).

16. Method according to claim 15, **characterized in that**, once the container (3) has been received by the holder (5), the positioning aid (15) is mounted directly on the container (3) or holder (5), wherein the positioning aid (15), in the course of being mounted, is brought into the predefined height position and/or circumferential position relative to at least one reference point (P) provided on the container (3) and/or holder (5), wherein the sensor (12) is then aligned with the positioning aid (15), or **in that** the positioning aid (15) is already mounted directly on the container (3) or holder (5) before the predefined height position and/or circumferential position is set, optionally even before the container (3) is received by the holder (5), wherein the positioning aid (15) is then brought into the predefined height position and/or circumferential position relative to at least one reference point (P) provided on the container (3) and/or holder (5), wherein the sensor (12) is then aligned with the positioning aid (15), or **in that** the positioning aid (15), before being mounted on the container (3) or holder (5), in particular once the container (3) has been received by the holder (5), is brought into a predefined position which, in the mounted state of the positioning aid (15), corresponds in each case to a specific height position and/or circumferential position relative to at least one reference point (P) provided on the container (3) and/or holder (5), wherein the positioning aid (15) is then mounted directly on the container (3) or holder (5), wherein the sensor (12) is then aligned with the positioning aid (15).

17. Method according to claim 15, **characterized in that** the positioning aid (15) is already mounted directly on the container (3) or holder (5) before the container (3) is received by the holder (5), wherein the sensor (12), once the container (3) has been received by the holder (5), is brought into the predefined height position and/or circumferential position relative to the positioning aid (15) and is aligned with the positioning aid (15).

## Revendications

1. Système de positionnement de capteur pour une installation de bioprocédés (1),
dans lequel un récipient (3), en particulier récipient à usage unique, est prévu pour la réception d'un milieu biologique, qui s'étend dans l'état installé dans la direction axiale (X) à partir d'une extrémité de récipient supérieure (3a) vers une extrémité de récipient inférieure (3b),
dans lequel un support (5) pour maintenir le récipient (3) dans l'état installé est prévu,
dans lequel un capteur (12) à fixer sur le récipient (3) est prévu,
**caractérisé en ce**
**qu'**un dispositif d'aide au positionnement (14) pour le positionnement du capteur (12) à fixer dans une position prédéfinie sur le récipient (3) est prévu,
**que** le dispositif d'aide au positionnement (14) présente une aide au positionnement (15) montée ou pouvant être montée directement sur le récipient (3) ou à côté du récipient (3) sur le support (5), qui peut être amenée dans différentes positions en hauteur et/ou circonférentielles par rapport à au moins un point de référence (P) prévu sur le récipient (3) et/ou support (5), et
**que** le dispositif d'aide au positionnement (14) présente un support de stockage technique ou support d'impression analogique (16) avec une liste d'affectation (17), dans laquelle respectivement une position en hauteur et/ou circonférentielle de l'aide au positionnement (15) est affectée à plusieurs cas d'utilisation de bioprocédés.

2. Système de positionnement de capteur selon la revendication 1, **caractérisé en ce que** le support de stockage ou d'impression (16) comprend un support de stockage électronique, magnétique, optique ou photographique, ou un support d'impression sous forme de papier ou sous forme de film.

3. Système de positionnement de capteur selon la revendication 1 ou 2, **caractérisé en ce que** respectivement au moins un, en particulier exactement un, point de référence (P) prévu sur le récipient (3) et/ou support (5) est affecté dans la liste d'affectation (17) aux plusieurs cas d'utilisation de bioprocédés, dans lequel respectivement une position en hauteur et/ou circonférentielle prédéfinie de l'aide au positionnement (15) est affectée au point de référence (P) respectif.

4. Système de positionnement de capteur selon l'une quelconque des revendications précédentes, **caractérisé en ce que** l'aide au positionnement (15) peut être disposée de manière fixe sur le récipient (3) ou support (5) dans son ensemble dans la direction axiale (X) dans différentes positions par rapport au point de référence (P) respectif, ou que l'aide au positionnement (15) peut être montée de manière fixe sur le récipient (3) ou support (5), dans lequel avant le montage de l'aide au positionnement (15) une partie (15a) de l'aide au positionnement (15) par ailleurs mobile par rapport au récipient (3) et/ou au support (5) et/ou à l'aide au positionnement (15) peut être disposée le long de l'aide au positionnement (15) par ailleurs dans différentes positions, qui dans l'état monté de l'aide au positionnement (15) correspondent respectivement à une position définie par rapport au point de référence (P) respectif, ou que l'aide au positionnement (15) est montée de manière fixe sur le récipient (3) ou support (5), dans lequel une partie (15a) de l'aide au positionnement (15) mobile par rapport au récipient (3) et/ou support (5) peut être disposée dans la direction axiale (X) dans différentes positions par rapport au point de référence (P) respectif.

5. Système de positionnement de capteur selon l'une quelconque des revendications précédentes, **caractérisé en ce que** l'aide au positionnement (15) peut être disposée de manière fixe sur le récipient (3) ou support (5) dans son ensemble dans la direction circonférentielle (U) dans différentes positions par rapport au point de référence (P) respectif, ou que l'aide au positionnement (15) peut être montée de manière fixe sur le récipient (3) ou support (5), dans lequel avant le montage de l'aide au positionnement (15) une partie (15a) de l'aide au positionnement (15) par ailleurs mobile par rapport au récipient (3) et/ou au support (5) et/ou à l'aide au positionnement (15) peut être disposée transversalement par rapport à l'aide au positionnement (15) par ailleurs dans différentes positions, qui dans l'état monté de l'aide au positionnement (15) correspondent respectivement à une position définie par rapport au point de référence (P) respectif, ou que l'aide au positionnement (15) est montée de manière fixe sur le récipient (3) ou support (5), dans lequel une partie (15a) de l'aide au positionnement (15) mobile par rapport au récipient (3) et/ou support (5) peut être disposée dans la direction circonférentielle (U) dans différentes positions par rapport au point de référence (P) respectif.

6. Système de positionnement de capteur selon l'une quelconque des revendications précédentes, **caractérisé en ce que** l'aide au positionnement (15) peut être reliée au récipient (3) et/ou au support (5) par liaison de matière, par coopération de formes et/ou à force.

7. Système de positionnement de capteur selon l'une quelconque des revendications précédentes, **caractérisé en ce que** l'aide au positionnement (15) fournit au moins un marquage (20), sur lequel le capteur (12) peut être orienté, de préférence que l'aide au positionnement (15) présente ou forme elle-même le au moins un marquage (20).

8. Système de positionnement de capteur selon l'une quelconque des revendications précédentes, **caractérisé en ce que** l'aide au positionnement (15) est un gabarit (21) pouvant être placé sur le récipient (3), qui peut être collé en particulier sur le récipient (3), ou que l'aide au positionnement (15) est un support de marquage (22) pouvant être placé sur le récipient (3) ou à côté du récipient (3) sur le support (5), présentant plusieurs marquages (20), en particulier sous forme d'un partage, de préférence un support de marquage (22) conçu sous la forme d'une barre ou bande, qui peut être fixé en particulier sur le récipient (3) et/ou support (5) ou peut être appliqué sur le récipient (3) et/ou support (5), de préférence qu'au moins un coulisseau (19) mobile entre les marquages (20) est disposé sur le support de marquage (22), ou que plusieurs marquages (20) apposés directement sur le récipient (3) et/ou support (5), en particulier sous forme d'un partage, sont prévus en tant qu'aide au positionnement (15).

9. Système de positionnement de capteur selon l'une quelconque des revendications précédentes, **caractérisé en ce que** l'aide au positionnement (15) est isolée du support (5) et/ou d'une porte (6) du support (5) ou est intégrée dans le support (5) et/ou une porte (6) du support (5), et/ou que le support de stockage ou d'impression (16) est séparé du récipient (3) et/ou support (5) ou monté, en particulier collé sur le récipient (3) et/ou support (5).

10. Système de positionnement de capteur selon l'une quelconque des revendications précédentes, **caractérisé en ce que** le récipient (3) présente un sachet pour bioprocédés (4) ou un récipient indéformable, de préférence pour un bioréacteur (2) ou un système de mélange pour bioprocédés, et/ou que le récipient (3) est un agitateur (9) intégré.

11. Système de positionnement de capteur selon l'une quelconque des revendications précédentes, **caractérisé en ce qu'**un ou plusieurs points de référence (P) sont formés sur le récipient (3) par l'extrémité de récipient supérieure ou l'inférieure (3a, 3b), par une soudure de sachet, par une saillie mécanique sur le récipient (3), par une saillie mécanique (23) sur un arbre d'agitation (9a) de l'agitateur (9) et/ou par une pièce de raccordement (7) sur le récipient (3), et/ou qu'un ou plusieurs points de référence (P) sont formés sur le support (5) par une arête (18a), en particulier une ouverture (18), et/ou par une saillie mécanique sur le support (5).

12. Système de positionnement de capteur selon l'une quelconque des revendications précédentes, **caractérisé en ce que** le capteur (12) est conçu sous la forme d'un capteur inductif, sous la forme d'un capteur capacitif, sous la forme d'un capteur optique, sous la forme d'un capteur ultrasonore, sous la forme d'un capteur à micro-ondes, sous la forme d'un capteur radar ou sous la forme d'un capteur pour la mesure de la conductivité électrique ou conductivité thermique, et/ou que le capteur (12) est conçu sous la forme d'un capteur de niveau de remplissage, de préférence sous la forme d'un interrupteur de niveau de remplissage ou sous la forme d'un capteur pour la mesure continue de niveau de remplissage et est en particulier un capteur de mousse.

13. Système de positionnement de capteur pour une installation de bioprocédés (1),
dans lequel un récipient (3), en particulier récipient à usage unique, est prévu pour la réception d'un milieu biologique, qui s'étend dans l'état installé dans la direction axiale (X) à partir d'une extrémité de récipient supérieure (3a) vers une extrémité de récipient inférieure (3b),
dans lequel un support (5) pour maintenir le récipient (3) dans l'état installé est prévu,
dans lequel un capteur (12) à fixer sur le récipient (3) est prévu,
**caractérisé en ce**
**qu'**un dispositif d'aide au positionnement (14) pour le positionnement du capteur (12) à fixer dans une position prédéfinie sur le récipient (3) est prévu,
**que** le dispositif d'aide au positionnement (14) présente une aide au positionnement (15) montée directement sur le récipient (3) ou à côté du récipient (3) sur le support (5), par rapport à laquelle le capteur (12) peut être amené dans différentes positions en hauteur et/ou circonférentielles, et
**que** le dispositif d'aide au positionnement (14) présente un support d'enregistrement technique ou support d'impression analogique (16) avec une liste d'affectation (17), dans laquelle respectivement une position en hauteur et/ou circonférentielle prédéfinie du capteur (12) est affectée à plusieurs cas d'utilisation de bioprocédés.

14. Installation de bioprocédés, en particulier bioréacteur (2) ou système de mélange pour procédés, avec un système de positionnement de capteur (13) selon l'une quelconque des revendications précédentes.

15. Procédé pour le positionnement d'un capteur (12) d'une installation de bioprocédés (1), en particulier au moyen d'un système de positionnement de capteur (13) selon l'une quelconque des revendications 1 à 14,
dans lequel un récipient (3), en particulier récipient à usage unique, est fourni pour la réception d'un milieu biologique,
dans lequel le récipient (3) est reçu par un support (5), qui porte le récipient (3), de telle sorte que le récipient (3) s'étend dans la direction axiale (X) à partir d'une extrémité de récipient supérieure (3a) vers une extrémité de récipient inférieure (3b),
**caractérisé en ce**
**qu'**après la réception du récipient (3) par le support (5) le capteur (12) est orienté sur une aide au positionnement (15) d'un dispositif d'aide au positionnement (14) et est fixé sur le récipient (3), dans lequel l'orientation s'effectue sur la base d'une position en hauteur et/ou circonférentielle prédéfinie, qui est affectée dans une liste d'affectation (17) d'un support de stockage technique ou support d'enregistrement analogique (16) à un parmi plusieurs cas d'utilisation de bioprocédés.

16. Procédé selon la revendication 15, **caractérisé en ce que** l'aide au positionnement (15) après la réception du récipient (3) par le support (5) est montée directement sur le récipient (3) ou support (5), dans lequel l'aide au positionnement (15) est amenée au cours de son montage par rapport à au moins un point de référence (P) prévu sur le récipient (3) et/ou support (5) dans la position en hauteur et/ou position circonférentielle prédéfinie, dans lequel le capteur (12) est ensuite orienté sur l'aide au positionnement (15) ou que l'aide au positionnement (15) déjà avant le réglage de la position en hauteur et/ou position circonférentielle prédéfinie, éventuellement également déjà avant la réception du récipient (3) par le support (5), est directement montée sur le récipient (3) ou support (5), dans lequel l'aide au positionnement (15) est ensuite amenée par rapport à au moins un point de référence (P) prévu sur le récipient (3) et/ou support (5) dans la position en hauteur et/ou position circonférentielle prédéfinie, dans lequel le capteur (12) est ensuite orienté sur l'aide au positionnement (15), ou que l'aide au positionnement (15) est amenée avant le montage sur le récipient (3) ou support (5), en particulier après la réception du récipient (3) par le support (5), dans une position prédéfinie, qui dans l'état monté de l'aide au positionnement (15) correspond respectivement à une position en hauteur et/ou position circonférentielle définie, par rapport à au moins un point de référence (P) prévu sur le récipient (3) et/ou support (5), dans lequel l'aide au positionnement (15) est ensuite montée directement sur le récipient (3) ou support (5), dans lequel le capteur (12) est ensuite orienté sur l'aide au positionnement (15).

17. Procédé selon la revendication 15, **caractérisé en ce que** l'aide au positionnement (15) avant la réception du récipient (3) par le support (5) est déjà montée directement sur le récipient (3) ou support (5), dans lequel le capteur (12) après la réception du récipient (3) par le support (5) est amené par rapport à l'aide au positionnement (15) dans la position en hauteur et/ou position circonférentielle prédéfinie et orienté sur l'aide au positionnement (15).
